# EUROPEAN PATENT SPECIFICATION

(11) **EP 0 548 834 B1**
(45) Date of publication and mention of the grant of the patent: **22.11.1995**
(21) Application number: 92121577.8
(22) Date of filing: 18.12.1992
(51) Int. Cl.: C07H 17/04, A61K 31/70

(54) **Stable hexahydrate of etoposide 4'-phosphate disodium salt**
Stabiles Hexahydrat von Etoposid-4'-Phosphat-Dinatriumsalz
Stabile hexahydrate d'étoposide 4'-phosphate sel disodium

(30) Priority: 23.12.1991 US 812597
(43) Date of publication of application: 30.06.1993
(73) Proprietor: Bristol-Myers Squibb Company, New York, N.Y. 10154 (US)
(72) Inventor: Gogate, Uday Shankar, East Syracuse, New York 13057 (US); Light, William Francis, East Syracuse, New York 13057 (US); Agharkar, Shreeram Narahari, Fayetteville, New York 13066 (US)
(74) Representative: VOSSIUS & PARTNER

(56) References cited:
- EP-A- 0 369 369
- GB-A- 2 207 674

## Description

The present invention relates to a crystalline hydrate of etoposide 4'-phosphate disodium salt.

Etoposide is an anticancer agent currently approved in the United States for the treatment of small cell lung cancer and refractory testicular tumor. Because etoposide is only sparingly soluble in water, an organic solvent or a mixture of organic solvents is required to prepare etoposide solution. The etoposide product for parenteral administration currently being marketed is contained in a multi-solvent system. The preparation of etoposide is disclosed in U.S. patent 3,408,441.

Etoposide 4'-phosphate disodium salt (I) is disclosed in United States Patent 4,904,768 as a prodrug form of etoposide. It has been shown to be as active as etoposide in in vivo antitumor assays.

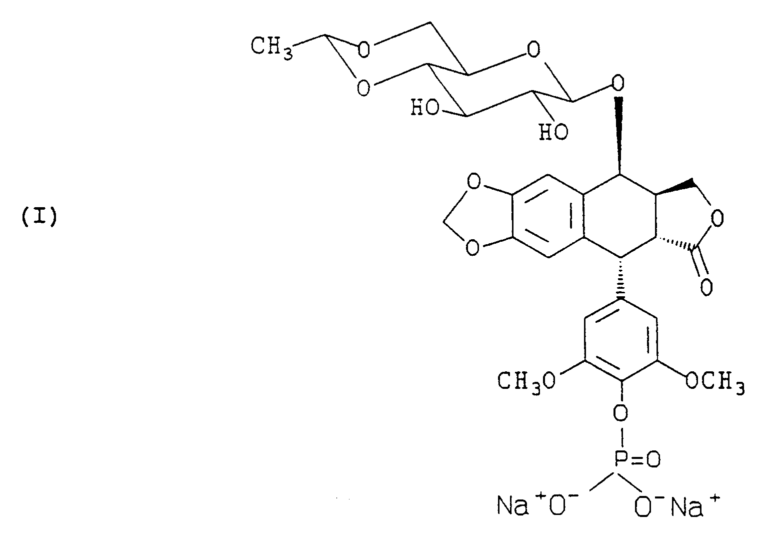

Whereas the solubility of etoposide in water is about 0.1 mg/ml, the disodium salt of etoposide 4'-phosphate exhibits water solubility of ≧100 mg/ml, thereby allowing the preparation of pharmaceutical formulations containing little or no organic solvent. The previously disclosed disodium salt of etoposide 4'-phosphate is a fluffy, amorphous material which is difficult to handle and which tends to be chemically unstable when stored. After 56 days storage at 30°C in sealed glass vials potency generally drops to about 85%.

In GB-A-2 207 674 the disodium salt of etoposide 4'-phosphate is described. Microscopic examination of the salt has revealed it to be a mixture of crystals and the amorphous form.

EP-A-0 369 369 relates to dihydrate crystals of an etoposide-2-dimethylamino compound HCL.

The present invention provides a stable crystalline etoposide 4'-phosphate disodium salt hydrate of formula (II): 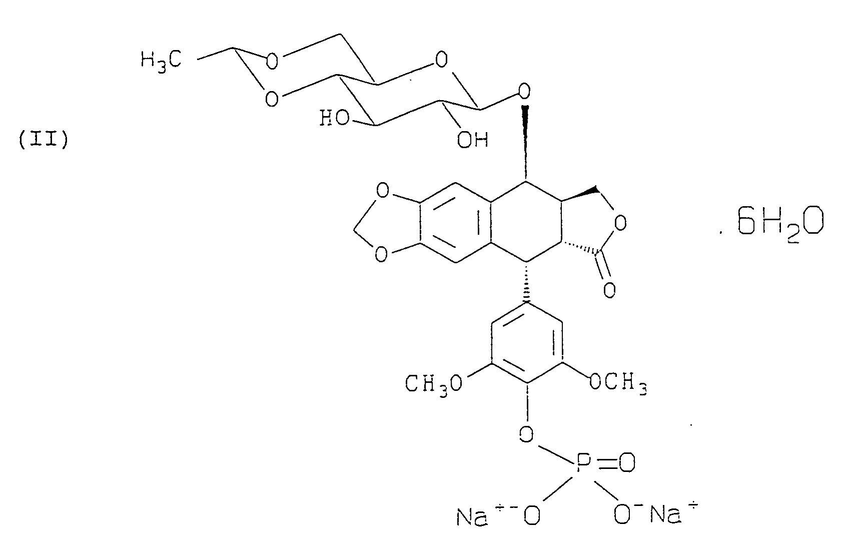
In one embodiment, the hexahydrate showed >99% potency after storage for 63 days at 30°C.

The present invention also provides a process for preparing the crystalline hexahydrate of etoposide 4'-phosphate disodium salt which comprises either the exposure of the anhydrous salt to high relative humidity for about 14 days or more or a procedure involving extracting and recrystallizing the hydrate from a suitable water/solvent mixture.

### BRIEF DESCRIPTION OF THE DRAWINGS

Figure 1 shows the proton NMR spectrum (D₂O, 200 MHz) of etoposide 4'-phosphate disodium hexahydrate.

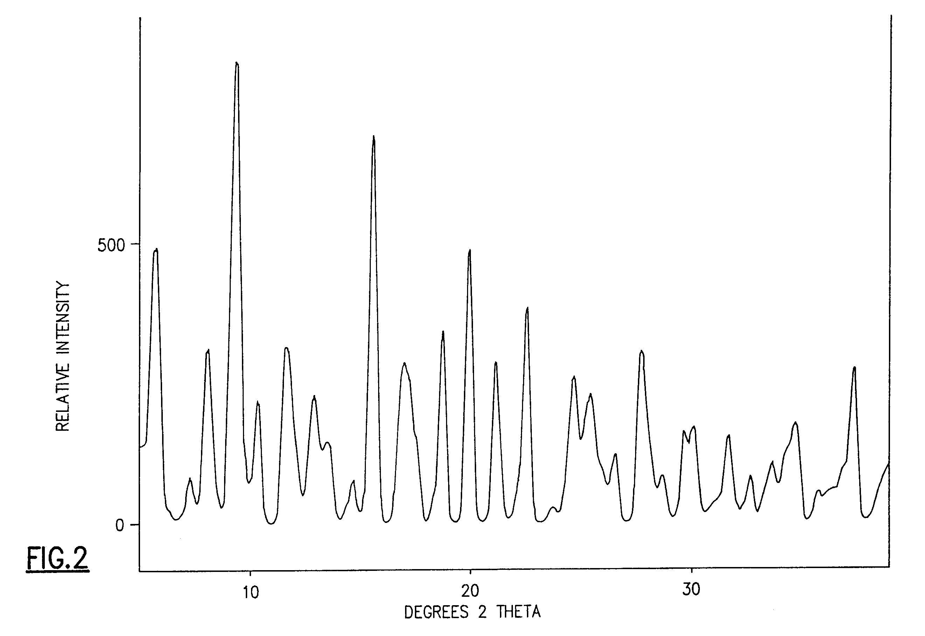

Figure 2 shows an X-ray powder diffraction pattern of etoposide 4'-phosphate disodium hexahydrate.

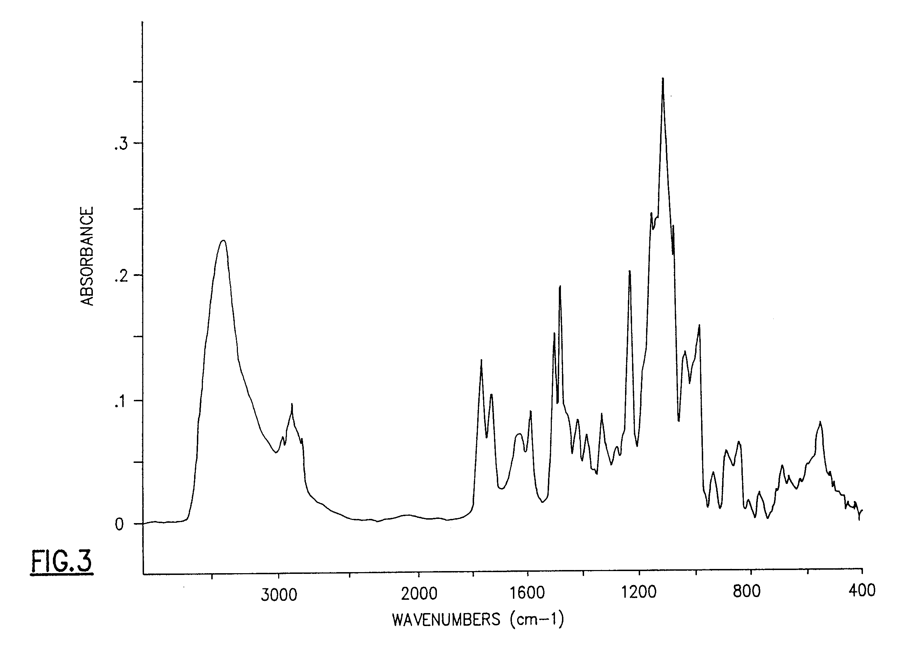

Figure 3 shows an infrared spectrum of the hexahydrate of the invention.

Etoposide 4'-phosphate disodium salt is a fluffy low density solid. Left in its anhydrous form, it slowly looses its potency, so that after 28 days at 37°C, only about 87% potency is left. The hexahydrate of this salt has 100% potency remaining after storage under the same conditions.

The advantages of the invention include the greater storage stability and ease of solution of the hydrated form. In addition, the relative ease with which the hydrate is produced makes its use in commercial products very attractive. One need not risk interaction with added stabilizers when formulating products based upon the hydrated salts.

Since the hydrate has the same basic chemical structure as the salt, it can be substituted for same in storage stable formulation to be administered to inhibit mammalian tumors.

The present invention provides a compound, compositions, and their use for inhibiting mammalian tumors.

For this purpose, the drug may be administered by conventional routes including, but not limited to, intravenous, intramuscular, intratumoral, intraarterial, intralymphatic, oral, buccal, nasal, ocular, and the like.

Thus, the present invention provides pharmaceutical compositions which comprise suitable amounts of the hexahydrate and of one or more pharmaceutically acceptable carriers. The antitumor compositions may be made up of any pharmaceutical form appropriate for the desired route of administration. Examples of such compositions include solid compositions for oral administration such as tablets, capsules, pills, powders and granules, liquid compositions for oral administration such as solutions, suspensions, syrups or elixirs and preparations for parenteral administration such as sterile solutions, suspensions or emulsions. They may also be manufactured in the form of sterile solid compositions which can be dissolved in sterile water, 0.9% sodium chloride solution or some other sterile injectable medium before use.

Optimal dosages and regimens for a given mammalian host can be readily ascertained by those skilled in the art. It will, of course, be appreciated that the actual dose used will vary according to the particular composition formulated, the particular compound used, the mode of application and the particular site, host and disease being treated. Many factors that modify the action of the drug will be taken into account including age, weight, sex, diet, time of administration, route of administration, rate of excretion, condition of the patient, drug combinations, reaction sensitivities and severity of the disease.

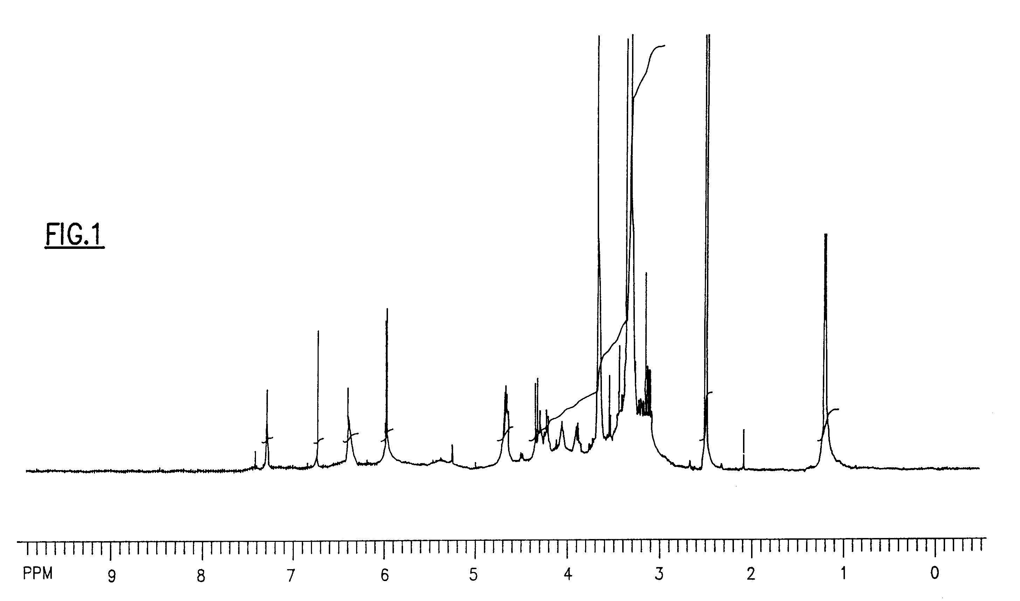

**Figure 1 was obtained using the following parameters**:

| Acquisition Parameters | |
|---|---|
| PULPROG | zg30 |
| NUCLEUS | 1H |
| SOLVENT | DMSO |
| AQ | 1,9988434 sec |
| FIDRES | 0.250145 Hz |
| DW | 61.0 usec |
| RG | 1024 |
| HL1 | 3 dB |
| D1 | 2.0000000 sec |
| P1 | 17.0 usec |
| RD | 0.0000000 usec |
| PW | 0.0 usec |
| DE | 76.3 usec |
| SF01 | 400.1378018 MHZ |
| SWH | 8196.74 Hz |
| TD | 32768 |
| NS | 32 |
| DS SI | 0 32768 |

| Processing parameters | |
|---|---|
| SI | 32768 |
| SF | 400.1362881 MHz |
| WDW | EM |
| SSB | 0 |
| LB | 0.30 Hz |
| GB | 0 |
| PC CX | 1.00 40.00 cm |

| NMR plot parameters | |
|---|---|
| CX | 40.00 cm |
| F1P | 10.000 ppm |
| F1 | 4001.36 Hz |
| F2P | -0.500 ppm |
| F2 | -200.07 Hz |
| PPMCM | 0.26250 ppm/cm |
| HZCM | 105.03577 Hz/cm |

### EXAMPLES

The following examples are for illustrative purposes only and should not be construed as limiting the scope of the invention which is defined solely by the claims appended to this application.

In the following examples, proton and carbon nuclear magnetic resonance (NMR) spectra (using CDCl₃ or D₂O as an internal reference) and phosphorous NMR spectra (using 85% aqueous H₃PO₄ as an external reference) were recorded on a Bruker WM360 spectrometer. Infrared (IR) spectra were determined on a Perkin-Elmer 1800 Fourier Transform Infrared Spectrophotometer. "Flash chromatography" refers to the method described by Still et al (Still, W.C.; Kahn, M.; Mitra, A.; J. Org. Chem., 1978, 43,2923) and was carried out using E. Merck silica gel (230-400 mesh). Reverse phase chromatography was carried out under a positive nitrogen pressure using C18 (octadecylsilane) bonded to silica gel (40-µm diameter, J.T. Baker supplier).

### EXAMPLE 1

### Etoposide 4'-Phosphate Disodium Salt

A magnetically stirred suspension of etoposide (2.30 g, 3.91 mmol) in dry acetonitrile (210 ml) was warmed to give a nearly complete solution. The solution was allowed to cool to room temperature, and N, N-diisopropylethylamine (2.36 ml, 13.5 mmol) was added. The mixture was then cooled to 0°C and POC1₃ (666 mg, 4.34 mmol) was added via syringe over 30 seconds. The mixture was allowed to slowly come to room temperature over 2-3 hours and stirring continued at room temperature for 63 hours. At the end of this period 20% by volume was removed and treated with diethylamine. The remainder was treated with a solution of sodium bicarbonate (6.0g, 71.4 mmol) in deionised H₂O (110 ml), the mixture was stirred at room temperature for 80 minutes, and then partitioned with ethyl acetate (350ml).

The organic layer was further extracted with deionized H₂O (1x50 ml) and the combined aqueous layers were washed with ethyl acetate (250 ml) and then subjected to a vacuum of 66.6 Pa (0.5 mm of Hg) at room temperature for 1 hour to remove dissolved solvents. The aqueous portion was then applied to a 4 cm diameter column containing 15 cm of octadecysilane bonded to silica gel which had been packed in methanol and equilibrated with H₂O. After all of the aqueous portion was applied, the column was eluted with H₂O (175 ml) to remove inorganic salts and then 4:1 H₂O:CH₃OH eluted the product. Concentration of the solvent at 66.6 Pa (0.5 torr) provided 744 mg (36%) of the pure title compound as a colorless solid. Alternatively, lyophilization provides the pure title compound as a very fluffy low denisty solid.
IR (KBr) 3426, 1775, 1593, 1486, 1337, 1239, 1191, 1122, 1078, 1034, 983, 927, 888, 876, 851, 840, 697, 684, 664, 547 cm⁻¹.
360 MHz ¹H NMR (D₂O)δ6.93(s,1H),6.27 (s, 2H), 5.93 (d,2H), 5.09(d, 1H, J=2.8 Hz), 4.83 (q, 1H, J=5.0 Hz), 4.68 (d,1H, J=7.9 Hz), 4.62 (d, 1H, J=5.7 Hz), 4.47-4.35 (m,2H), 4.24 (dd,1H, J=4.4 and 10.4 Hz), 3.64 (s,6H (s,6H), 3.68-3.52(m,3H), 3.44-3.30 (m,3H), 3.17-3.07 (m,1H), 1.31 (d,3H, J=5.0 Hz).
90MHz ¹³C NMR (D₂O)δ178.5, 151.8, 148.1, 146.1, 135.0, 132.6, 130.9, 127.4, 109.9, 109.5, 107.4, 101.3, 100.4, 99.6, 79.2, 73.7, 72.7, 72.2, 69.1, 67.1, 65.4, 55.6, 42.8, 40.3, 37.5, 18.8.
146 MHz ³¹P NMR (D₂O) δ3.79.
Mass spectrum (FAB), m/e, 713 (M⁺+H). C₂₉H₃₁Na₂O₁₆P requires M⁺, 712.
Anal. Calcd. for C₂₉H₃₁Na₂O₁₆P: C, 48.89; H, 4.39; Na, 6.45. Found*: C, 48.72; H, 4.56; Na, 6.56.
*Adjusted for 8.16% H₂O determined by Karl Fischer analysis.

### EXAMPLE 2

### Stability of Anhydrous Etoposide 4'-Phosphate Disodium Salt

A 0.01 g. sample of the anhydrous salt produced in accordance with example 1 was placed in a type I flint glass vial, stoppered, sealed, and stored for 56 days at 30°C. Only 85% potency remained.

Microscopic examination of the anhydrous salt revealed that it was a mixture of needle-shaped crystals and amorphous forms.

### EXAMPLE 3

### Hexahydrate of Etoposide 4'-Phosphate Disodium Salt

### Method A: Exposure to high (≧ 80%) relative humidity

A sample (0.01 g) of the salt produced in accordance with Example I was exposed to 80% relative humidity at 37°C for 28 days. The anhydrous sample exposed to humidified air showed a significant increase in chemical stability compared to the control sample (see Table 1).

The moisture level in the exposed sample increased from 8.9% to 13.7%, suggesting formation of a stable hexahydrate crystalline form (theoretical KF = 13.2%).

**TABLE 1**

| **Chemical Stability of Etoposide 4'-Phosphate Disodium salt** | | |
|---|---|---|
| **Storage Conditions 28 Days at** | **% KF** | **% Remaining** |
| Anhydrous; 37°C (control) | 8.9 | 87.4 |
| Hexahydrate (formed in situ): 80% Relative Humidity, 37°C | 13.7 | 100.0 |
| A stable hexahydrate was also obtained by exposure to 87% relative humidity (25°C) for 28 days. The exposed sample exhibited birefringence when examined under microscope, indicating crystalline nature of the sample. | | |

### Method B

Chemically pure hexahydrate was obtained by the reaction of etoposide 4'-phosphate with sodium ethyl hexanoate, followed by extraction with methylene chloride and recrystallization from water-acetone mixture. The phosphate (4.1 g) was dissolved in 82 mL of acetone. To this was added sodium ethyl hexanoate (2.3 g) dissolved in 41 mL of acetone. The suspension formed was stirred for 2.5 hours and then filtered through Fisher P8 filter paper. The residue on the filter paper was washed with two 10-mL acetone aliquots. The washed material was dissolved in 16 mL water and filtered through a Rainin Nylon-66 membrane (0.45 µm).

The filtrate was shaken vigorously with an equal volume of methylene chloride and the mixture was allowed to stand for 15 minutes. The aqueous layer was separated and 16 mL of acetone was added to it. The mixture was allowed to stand for one hour when a white, crystalline material separated. This mixture was stirred for an additional 16 hours and then filtered. The solid on the filter paper was dried under vacuum 4.123 kPa (31 mm of Hg) at 23°C for 6 hours.

This material was observed to be thermally more stable than the original salt prepared in Example 1 supra (see Table 2), suggesting the presence of a stable crystalline form. The Karl Fischer moisture value (ie., 13.8%) for this material suggested that it may be present as hexahydrate crystals (theoretical KF = 13.2%). Examination of the sample using microscopy and differential scanning calorimetry (Table 3) indicated that the synthesized material has a physical form similar to that of the sample exposed to artificially humidified air.

The elemental analysis of the synthesized sample agrees well with the molecular formula (C₂₉H₄₃O₂₂Na₂P) for Etoposide 4'-Phosphate Disodium salt hexahydrate.

| | | | |
|---|---|---|---|
| Theoretical: | C,42.45; | H, 5.28; | P, 3.77. |
| Found: | C, 42.38; | H, 5.10; | P, 3.63. |

**Table 2**

| **Storage Stability of Anhydrous and Hydrated Samples of Etoposide 4'-Phosphate Disodium Salt** | | |
|---|---|---|
| Sample | Storage Period at 50°C, in sealed glass vials (weeks) | % Remaining |
| Anhydrous Etoposide 4'-Phosphate Disodium Salt | 8 | 61.6 |
| Crystalline disodium Etoposide 4'-Phosphate* | 9 | 99.4 |

| | | |
|---|---|---|
| * Prepared by reaction of Etoposide 4'-Phosphate with sodium ethyl hexanoate, followed by extraction and recrystallization. | | |

Table 3 shows a comparison of the calorimetric properties of the hydrate made via Methods A and B. The results suggest that Methods A and B produce the same form of hexahydrate.

**Table 3**

| **Differential Scanning Calorimetric Examination of Hydrate Samples** | | |
|---|---|---|
| **Sample** | Endotherm Peak (°C) | Onset of Endotherm (°C) |
| Hexahydrate Method A | 157.0 | 140.4 |
| Hexahydrate Method B | 157.9 | 133.3 |

The values presented in Table 3 were derived via thermal analysis of samples using Perkin Elmer Series 7 system. 1 mg samples were heated at the rate of 10°C per minute and the resulting heat flow was measured.

## Claims

1. A stable crystalline hexahydrate of etoposide-4'-phosphate disodium salt having the formula

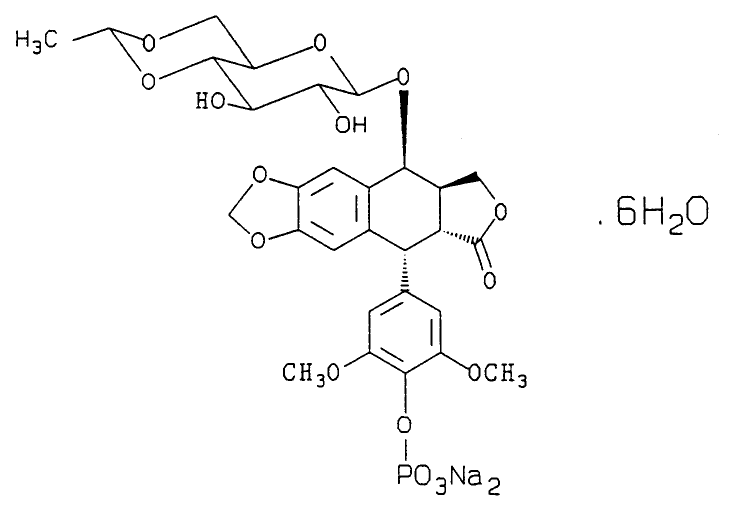

2. A process for preparing the crystalline hexahydrate of etoposide-4'-phosphate disodium salt which comprises reacting etoposide 4'-phosphate with a source of sodium ions, extracting with a suitable solvent, and recrystallizing it from an aqueous mixture which contains a second diluent.

3. The process of claim 2 wherein the solvent used in the extration step is methylene chloride.

4. The process of claim 3 wherein the aqueous mixture comprises water and acetone.

5. A pharmaceutical preparation containing a pharmaceutically acceptable carrier and the crystalline hexahydrate of etoposide-4'-phosphate disodium salt as defined in claim 1.

6. A process for preparing the crystalline hexahydrate of etoposide-4'-phosphate disodium salt which comprises exposing the anhydrous salt to ≧80% relative humidity for at least 14 days.

## Patentansprüche

1. Stabiles kristallines Hexahydrat von Etoposid-4'-phosphat Dinatriumsalz mit der Formel

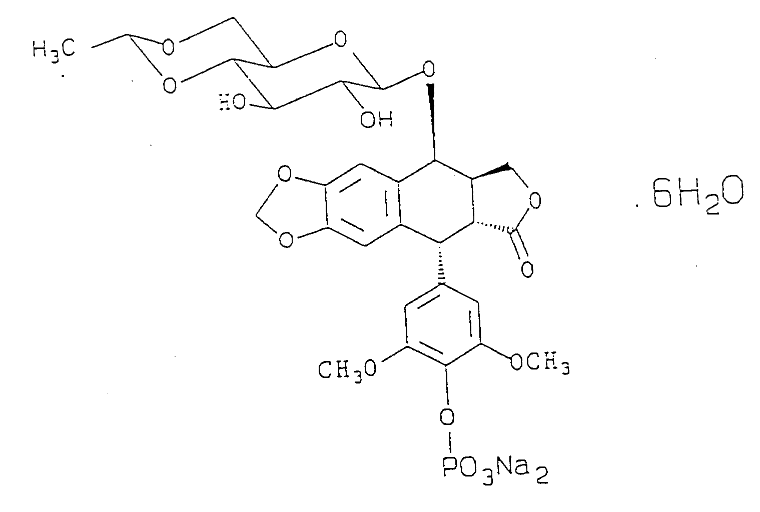

2. Verfahren zur Herstellung des kristallinen Hexahydrats von Etoposid-4'-phosphat Dinatriumsalz, das die Umsetzung von Etoposid-4'-phosphat mit einer Natriumionen-Quelle, Extraktion mit einem geeigneten Lösungsmittel und Umkristallisation aus einem wässrigen Gemisch, das ein zweites Verdünnungsmittel enthält, umfasst.

3. Verfahren nach Anspruch 2, wobei das bei dem Extraktionsschritt verwendete Lösungsmittel Methylenchlorid ist.

4. Verfahren nach Anspruch 3, wobei das wässrige Gemisch Wasser und Aceton umfasst.

5. Arzneimittel, das eine pharmazeutisch verträgliche Trägersubstanz und das kristalline Hexahydrat von Etoposid-4'-phosphat Dinatriumsalz, wie in Anspruch 1 definiert, enthält.

6. Verfahren zur Herstellung des kristallinen Hexahydrates von Etoposid-4'-phosphat Dinatriumsalz, das die Einwirkung von ≧80%iger relativer Feuchte für wenigstens 14 Tage auf das wasserfreie Salz umfasst.

## Revendications

1. Hexahydrate cristallin stable du sel disodique d'étoposide-4'-phosphate ayant la formule

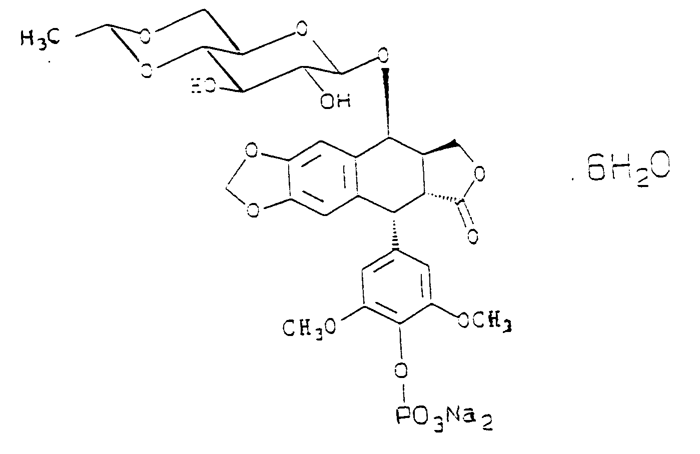

2. Procédé de préparation de l'hexahydrate cristallin du sel disodique d'étoposide-4'-phosphate qui comprend la mise en réaction de l'étoposide-4'-phosphate avec une source d'ions sodium, l'extraction avec un solvant adéquat, et sa recristallisation à partir d'un mélange aqueux qui contient un second diluant.

3. Procédé selon la revendication 2 dans lequel le solvant utilisé dans l'étape d'extraction est le chlorure de méthylène.

4. Procédé selon la revendication 3 dans lequel le mélange aqueux comprend de l'eau et de l'acétone.

5. Préparation pharmaceutique comprenant un support pharmaceutiquement acceptable et l'hexahydrate cristallin du sel disodique d'étoposide-4'-phosphate comme défini à la revendication 1.

6. Procédé de préparation de l'hexahydrate cristallin du sel disodique d'étoposide-4'-phosphate qui comprend l'exposition du sel anhydre à une humidité relative ≧ 80% pendant au moins 14 jours.
